# EUROPÄISCHE PATENTSCHRIFT

(11) **EP 2 758 759 B1**
(45) Veröffentlichungstag und Bekanntmachung des Hinweises auf die Patenterteilung: **29.11.2017**
(21) Anmeldenummer: 12774948.9
(22) Anmeldetag: 19.09.2012
(51) Int. Cl.: G01K 11/32, A61B 5/01, G01K 13/00

(54) **MEDIZINTECHNISCHE SYSTEME MIT EINEM TEMPERATURSENSOR ODER EINER TEMPERATURMESSVORRICHTUNG**
MEDICAL SYSTEMS WITH A TEMPERATURE SENSOR OR A TEMPERATURE MEASURING APPARATUS
SYSTÈME TECHNIQUE MÉDICAL COMPRENANT UN CAPTEUR DE TEMPÉRATURE OU UN DISPOSITIF DE MESURE DE TEMPÉRATURE

(30) Priorität: 19.09.2011 DE 102011053755
(43) Veröffentlichungstag der Anmeldung: 30.07.2014
(73) Patentinhaber: Aesculap AG, 78532 Tuttlingen (DE)
(72) Erfinder: KELLER, Anton, 78589 Dürbheim (DE); EICK, Stefan, 78532 Tuttlingen (DE); MASER, Thomas, 78658 Zimmern ob Rottweil (DE); ROTHWEILER, Christoph, 78166 Donaueschingen (DE); LANGEN, Sebastian, 67459 Böhl-Iggelheim (DE); HIBST, Raimund, 89155 Erbach (DE); FUGGER, Oliver, 89075 Ulm (DE); RUSS, Detlef, 71272 Renningen (DE)
(74) Vertreter: Winter, Brandl, Fürniss, Hübner, Röss, Kaiser, Polte - Partnerschaft mbB
(86) Internationale Anmeldenummer: PCT/EP2012/068401
(87) Internationale Veröffentlichungsnummer: WO 2013/041550

(56) Entgegenhaltungen:
- DE-A1- 3 902 001
- US-A- 4 626 110
- US-A- 5 304 809
- US-A1- 2005 085 806
- US-A1- 2008 125 767

## Beschreibung

Die Erfindung betrifft Temperatursensoren für medizintechnische Systeme, Temperaturmessvorrichtungen, die solche Temperatursensoren enthalten sowie die medizintechnischen Systeme, die diese Temperatursensoren bzw. Temperaturmessvorrichtungen umfassen.

Die Temperaturmessung bei medizinischen Behandlungen und Therapien, insbesondere auch in der Chirurgie, sind in vielfältiger Weise von Bedeutung, insbesondere auch, um einen Therapie- oder Behandlungsfortschritt erkennen zu können, diesen zu steuern und gegebenenfalls zu beenden.

Mit den herkömmlichen Temperatursensoren ist eine unbeeinflusste Temperaturmessung parallel zu laufenden Therapien oder Behandlungsmaßnahmen, insbesondere auch chirurgische Eingriffen, oft unmöglich, da eine Vielzahl von Umgebungsfaktoren die Temperaturmessung beeinflussen und so den eigentlich zu bestimmenden Temperaturwert verfälschen.

Beispielsweise spielen sowohl elektrische Störungen bei HF-Chirurgie-Geräten als auch die direkte Erwärmung der Sensoren bei der Erwärmung durch einen Laserstrahl als Störfaktoren eine wesentliche Rolle.

Bei elektrochirurgischen Eingriffen erfolgt derzeit eine Steuerung des Vorgangs durch die Messung der elektrischen Impedanz der behandelten Gewebestruktur, die mit dem Wassergehalt der Gewebestruktur korreliert. Während der HF-Bestromung kann allerdings die Temperaturmessung nicht stattfinden.

Auch bei Anwendung von Lasern ist eine direkte Temperaturmessung mit Thermoelementen oder Thermistoren aufgrund der Eigenabsorption von Laserenergie durch diese Elemente nicht möglich.

Die vorgenannte Impedanzmessung kann nur als indirekte Größe die Reaktion der Gewebestruktur auf den thermischen Reiz abbilden. Eine merkliche Veränderung der Impedanz tritt allerdings nur dann auf, wenn eine nennenswerte Menge des Gewebewassers verdampft wird. Die Gewebestruktur selbst wird aber bereits schon bei deutlich niedrigeren Temperaturen beeinflusst und verändert. So werden beispielsweise Konformationsänderungen im Kollagen bereits bei Temperaturen von ca. 70°C erreicht. Dabei spielt neben der Temperatur auch die Einwirkdauer des Temperaturreizes eine wesentliche Rolle. Solche Prozesse werden, wie andere chemische Reaktionen auch, durch Arrhenius-Koeffizienten beschrieben.

Darüber hinaus ist die Impedanzmessung in hohem Maße von der Größe des bei der Messung relevanten Bereichs der behandelten Gewebestruktur sowie von dem Abstand und Anpressdruck der Elektroden abhängig.

Des Weiteren wurde in der WO 2009/005850 A1 die Messung der Lichttransmission zur Beurteilung des Zustandes des behandelten Gewebes herangezogen. Dieses Messverfahren ist störungsanfällig bezüglich Verschmutzungen und oft nicht ausreichend aussagekräftig im Hinblick auf den von dem beobachteten Gewebe erreichten Zustand.

Die US 4 626 110 A misst beispielsweise die Temperatur eines Gewebes, welches mittels Hypothermie behandelt wird, indem ein Sensor, der auf optischer Basis arbeitet, in ein Gewebevolumen implantiert wird, um eine von aussen, über einen Ultraschallerzeuger, bewirkte, kontinuierliche Gewebeerwärmung zu messen.

Die US 2005/085806 A1 offenbart eine elektrochirurgische Sonde zur Durchführung von Elektrochirurgie, wobei die Sonde eine aktive Elektrode und eine Rückelektrode aufweist, die durch ein isolierendes Element getrennt sind. Weiterhin sind Mittel vorhanden, zum Erfassen und Weiterleiten von pH-Konzentrationen in der Nähe der aktiven Elektrode und der Rückelektrode.

Die US 2008/125767 A1 offenbart eine Verbesserung der Messung eines elektrischen Thermokopplers, der in ein zangenartiges Instrument eingebaut ist. Die Messung der Gewebetemperatur wird über eine an den Thermokoppler angepasste Messschaltung ausgewertet.

Aufgabe der vorliegenden Erfindung ist es, einen Temperatursensor bzw. eine Temperaturmessvorrichtung vorzuschlagen, die eine direkte Temperaturmessung ermöglicht, insbesondere auch während der Behandlung, insbesondere auch mit HF-Chirurgie-Geräten.

Diese Aufgabe wird erfindungsgemäß durch einen Temperatursensor bzw. eine diesen enthaltende Temperaturmessvorrichtung der Ansprüche 1 bzw. 7 gelöst.

Die vorliegende Erfindung nutzt den Effekt, dass die Quantenausbeute, vorzugsweise die Lumineszenzquantenausbeute, insbesondere auch die Fluoreszenzquantenausbeute, temperaturabhängig ist und so ein Maß für die Temperatur eines Lumineszenzanregbaren Sensorelements bietet.

Die Temperaturbestimmung einer Probe anhand der Temperaturabhängigkeit der Fluoreszenzquantenausbeute von fluoreszendierenden Mitteln ist an sich aus der WO 03/012468 A1 bekannt. Dort wird vorgeschlagen, auf die Oberfläche elektronischer Bauelemente, deren Temperatur durch modulierte elektrische Energie beeinflusst wird, ein fluoreszendierendes Mittel aufzubringen. Die von der mit Anregungslicht beaufschlagten, so modifizierten Probenoberfläche abgestrahlte Fluoreszenz wird von einer Bildaufnahmevorrichtung aufgenommen und mit der Modulationsfrequenz der elektrischen Energie phasengekoppelt ausgewertet.

Im Gegensatz zu diesem herkömmlichen Verfahren arbeitet die vorliegende Erfindung bezüglich des Lumineszenzmediums und des Lichtaufnahmeelements in Form des Lichtleiters mit einem geschlossenen System in dem Sinne, dass die Beobachtung der Lumineszenzquantenausbeute unbeeinflusst von sonstigen Umgebungseinflüssen gelingt und damit erst in vielerlei medizintechnischen Systemen mit Erfolg einsetzbar ist.

Die vorliegende Erfindung benötigt für die genaue Bestimmung der Temperatur der beobachteten Probe keine aufwändige Messtechnik und lässt insbesondere die Temperaturmessung an Probenoberflächen zu, die typischerweise einer optischen Beobachtung nicht zugänglich sind.

Da der erfindungsgemäße Temperatursensor optisch mit einem Lichtleiter gekoppelt ist, der das Licht zum lumineszierenden Medium zuführt bzw. die Lumineszenz des Mediums ableitet, unabhängig von den optischen Gegebenheiten der Umgebung des Temperatursensors, beeinträchtigen Verschmutzungen des Sensorelements in einer Operationsumgebung des Messergebnis nicht.

Die erfindungsgemäß verwendete Messung der Temperatur im Rahmen der Bestimmung des Lumineszenzquantenausbeute ist darüber hinaus unempfindlich gegenüber elektrischen Störungen, die beispielsweise beim Betrieb von HF-Chirurgie-Geräten auftreten können. Selbst beim parallelen Arbeiten mit Laserlicht im Rahmen einer therapeutischen Behandlung findet keine Störung statt, da die Lichtfrequenzen für die Laserbehandlung einerseits und die Anrerungswellenlänge bzw. die Lumineszenzwellenlänge des Sensorelements andererseits jeweils so gewählt werden können, dass keine Beeinflussung der Messung der Lumineszenzquantenausbeute stattfindet.

Darüber hinaus kann das Sensorelement mit dem zur Lumineszenz anregbaren Medium sehr klein gehalten werden, so dass die Wärmekapazität des Sensorelements als solches vernachlässigbar ist und sich momentan Temperaturänderungen der umgebenden Gewebestruktur über die Lumineszenzmessung nachverfolgen lassen.

Mit der erfindungsgemäß möglichen direkten Temperaturmessung kann bereits während der Anwendung von hochfrequentem Strom bzw. Laserlicht der Zustand des Gewebes charakterisiert und ein angestrebter Versiegelungsprozess von Gewebestrukturen kontrolliert werden. Die Berechnung des Gewebezustandes kann dann über die zuvor erwähnten Arrhenius-Koeffizienten realisiert werden.

Die erfindungsgemäßen Temperatursensoren bzw. Temperaturmessvorrichtungen lassen sich somit sowohl bei Hochfrequenz-Anwendungen als auch bei Laseranwendungen oder anderen Energieformen zum Erwärmen von Gewebestrukturen verwenden. Die Temperaturmessung mit Hilfe der erfindungsgemäßen Temperatursensoren bzw. der damit ausgerüsteten Temperaturmessvorrichtungen ist darüber hinaus unabhängig von der Größe der behandelten Gewebestruktur und dem Elektrodenabstand.

Bei bevorzugten erfindungsgemäßen Temperatursensoren ist ein gemeinsamer Leiter zum Zuführen von Licht mit der Anregungswellenlänge einerseits und zum Aufnehmen und Ableiten von Licht mit der Lumineszenzwellenlänge andererseits vorhanden. Weiter bevorzugt ist das Sensorelement direkt mit dem oder den Lichtleiter(n) verbunden.

Die Sensorelemente für die erfindungsgemäßen Temperatursensoren können das zur Lumineszenz anregbare Medium in einer das Medium umgebenden Hülle umfassen, die insbesondere starr ausgebildet sein kann. Die Hülle kann optisch durchlässig oder opak, beispielsweise nach innen reflektierend, ausgebildet sein.

Das zur Lumineszenz anregbare Medium kann ein fließfähiges Medium, insbesondere ein Gel, sein.

Besonders bevorzugt sind zur Lumineszenz anregbare Medien in Form von Festkörpern, insbesondere Kristalle, die in Festkörperlasern Verwendung finden. Zu denen sind hier Rubin, Saphir, dotierte Yttrium-Aluminium-Granat (YAG)-Kristalle, beispielsweise ER:YAG, ND:YAG, YB:YAG sowie Alexandrit zu zählen.

Besondere Vorteile dieser Materialien sind deren hohe Wärmeleitfähigkeit, Temperaturresistenz und mechanische Festigkeit.

Andererseits lassen sich Feststoffe in Form von mit Fluoreszenzfarbstoffen funktionalisierten Kunststoffen wie z.B. PEEK als anregbares Medium einsetzen. Vorteilhaft bei den funktionalisierten Kunststoffmaterialien sind deren vielfältige Verfügbarkeit, einfache Verarbeitbarkeit sowie die geringen Materialkosten. Insbesondere bei PEEK kommen die breite Verwendung in der Medizintechnik und die gute Temperaturbeständigkeit hinzu.

Des Weiteren eignen sich mit Fluoreszenzfarbstoffen funktionalisierte temperaturbeständige auch andere Kunststoffe, z.B. Epoxidharze.

Bei mit Fluoreszenzfarbstoffen funktionalisierten Kunststoffmaterialien kann das Kunststoffmaterial aus einer großen Anzahl verfügbarer Materialien, die zudem auf die jeweilige Anwendung hin noch angepasst werden können, ausgewählt werden.

Sind die erfindungsgemäßen Temperatursensoren mit einer starren Hülle, die das fluoreszierende Medium umgibt, ausgestattet oder als Festkörper wie beschrieben ausgebildet, dann können die Temperatursensoren selbst auch als Abstandhalter bei HF-Anwendungen dienen, die die Elektroden auf einem vorgegebenen Abstand halten, um einen Kurzschluss bei der Bestromung der Elektroden zu vermeiden.

Bei einer solchen Doppeiverwendung als Temperatursensor und Abstandhalter findet gleichzeitig die Temperaturmessung so nah wie möglich am Ort des Energieeintrags statt, d.h. in direktem Kontakt mit dem behandelten Volumen der Gewebestruktur. Die Abstandhalter bzw. die Sensorelemente lassen so unmittelbar die Beobachtung der Temperaturänderung in der Umgebung zu.

Die erfindungsgemäßen Temperaturvorrichtungen für medizintechnische Systeme umfassen neben den bereits im Detail beschriebenen Temperatursensoren zusätzlich eine die Anregungswellenlänge abstrahlende Lichtquelle, insbesondere einen Laser, sowie einen Lumineszenzdetektor, beispielsweise eine Photozelle.

Bevorzugte Temperaturmessvorrichtungen beinhalten mehrere Temperatursensoren, die in Form einer zwei- oder dreidimensionalen Matrix in einer Halterung in vorgegebenem Abstand zueinander aufgenommen und angeordnet sind. Aufgrund der möglichen kleinen Abmessungen der Sensorelemente der erfindungsgemäßen Temperatursensoren lassen sich diese in viele herkömmliche Bauteile chirurgischer Instrumente und medizintechnischer Systeme integrieren, wobei die Bauteile dann als Halterung dienen können.

Die Erfindung betrifft schließlich medizintechnische Systeme, die einen oder mehrere der erfindungsgemäßen Temperatursensoren umfassen oder eine Temperaturmessvorrichtung wie zuvor beschrieben.

Bevorzugte medizintechnische Systeme sind als chirurgisches System, insbesondere als Ultraschall-, laser- oder elektrochirurgisches System ausgebildet.

Bevorzugte medizintechnische Systeme der vorliegenden Erfindung zeichnen sich dadurch aus, dass das chirurgische System eine Vorrichtung zum Schneiden, zur Dissektion, zur Koagulation, zur Versiegelung und/oder zur Verbindung von Gewebestrukturen eines Patienten umfasst.

Aufgrund der Verwendung der erfindungsgemäßen Temperatursensoren ist es dabei möglich, die Veränderung der Gewebestrukturen sehr viel präziser und früher zu erfassen als dies mit der herkömmlichen Impedanzmessung der Fall ist, da der Wassergehalt der behandelten Gewebestruktur keinen direkten Einfluss auf die Temperaturmessung nimmt.

Da die erfindungsgemäßen Temperatursensoren bei der entsprechenden Auswahl der Sensorelemente aus mechanisch und thermisch hoch resistenten Materialien direkt an der Gewebestruktur, die behandelt wird, appliziert werden können, ergibt sich so auch eine direkte Temperaturmessung, die der im Stand der Technik alternativ empfohlenen Messung der Infrarotstrahlung, die von einer behandelten Gewebestruktur ausgeht, weit überlegen ist. Bezug genommen werden kann diesbezüglich etwa auf die US 2007/0179484 A1.

Häufig weisen in erfindungsgemäßen medizintechnischen Systemen zur Behandlung von Gewebestrukturen ein Applikatorwerkzeug mit zwei Applikatorelementen, insbesondere in Form von so genannten Applikatorbranchen auf, welche gegeneinander beweglich und aus einer offenen Ruhestellung in eine geschlossene Arbeitsstellung überführbar sind. Hierbei wird dann vorzugsweise in mindestens einem der Applikatorelemente bzw. mindestens einer der Applikatorbranchen einer der erfindungsgemäßen Temperatursensoren angeordnet. Die Applikatorelemente, insbesondere wenn sie Teil eines HF-Chirurgie-Gerätes sind, weisen einen Abstandhalter auf, der die Applikatorelemente in der Arbeitsstellung in einem vorgegebenen Abstand zueinander hält, wobei dann der Abstandhalter insbesondere einen Temperatursensor der vorliegenden Erfindung umfasst. Insbesondere kann der Temperatursensor bzw. dessen Sensorelement selbst als Abstandhalter ausgebildet werden.

Bevorzugte medizintechnische Systeme der vorliegenden Erfindung umfassen einen Schutzmechanismus, welcher die Aktivierung des oder der erfindungsgemäßen Temperatursensoren blockiert, solange die Applikatorelemente nicht in einer vorgegebenen Applikationsstellung, insbesondere der Arbeitsstellung, angeordnet sind. Dies vermeidet, dass von den Temperatursensoren des medizintechnischen Systems unbeabsichtigt Licht in die Umgebung ausgestrahlt wird. Dies ist von besonderer Bedeutung, wenn zur Lumineszenzanregung ein Laser als Lichtquelle verwendet wird.

Der vorgenannte Schutzmechanismus weist vorzugsweise ein Schaltelement, insbesondere einen optischen, elektrischen oder mechanischen Kontaktgeber, auf, der die Stellung der Applikatorelemente überwacht.

Der Schutzmechanismus kann darüber hinaus einen separaten Temperatursensor umfassen, der die Temperatur mindestens eines der Applikatorelemente misst, so dass über diesen Temperatursensor geprüft werden kann, ob die Applikatorelemente bereits an einer Gewebestruktur anliegen, die typischerweise Körpertemperatur aufweist.

Wie bereits erwähnt, sind die erfindungsgemäßen medizintechnischen Systeme vorzugsweise mit einem HF-Generator ausgerüstet, einer erfindungsgemäßen Temperaturmessvorrichtung bzw. -sensor, einer Vorrichtung zur Auswertung eines Temperaturverlaufs und/oder einer Vorrichtung zum Regeln oder Steuern einer Systemfunktion in Abhängigkeit von dem mittels Temperatursensor gemessenen Temperaturwert.

Bei weiter bevorzugten medizintechnischen Systemen der vorliegenden Erfindung sind die Applikatorelemente mit einem strukturierten Oberflächenbereich ausgerüstet, der ein besonders sicheres Greifen einer Gewebestruktur ermöglicht. Insbesondere vermeidet dies bei der therapeutischen Behandlung ein unbeabsichtigtes Verschieben der zu behandelnden Gewebestruktur gegenüber den Applikatorelementen.

Als strukturierte Oberflächen eignen sich insbesondere gitterartig strukturierte Oberflächen.

Weiter bevorzugte medizintechnische Systeme weisen darüber hinaus eine Dosiervorrichtung für ein Primermaterial auf, wobei dies insbesondere in Form eines kollagen- oder gelatinebasierenden Materials bereitgestellt werden kann.

Diese und weitere Vorteile der Erfindung werden im Folgenden anhand der Zeichnung noch näher erläutert. Es zeigen im Einzelnen:
- Figur 1: einen Temperatursensor der vorliegenden Erfindung;
- Figuren 2A und 2B: den Temperatursensor der Figur 1 in zwei verschiedenen Sensoranordnungen;
- Figur 3: eine schematische Darstellung des Wirkungsprinzips des erfindungsgemäßen Temperatursensors;
- Figur 4: die Temperaturabhängigkeit der Fluoreszenzquantenausbeute eines erfindungsgemäßen Temperatursensors;
- Figur 5: ein medizintechnisches System der vorliegenden Erfindung; und
- Figur 6: einen Teil des medizintechnischen Systems der Figur 5 in vergrößerter und detaillierter Darstellung.

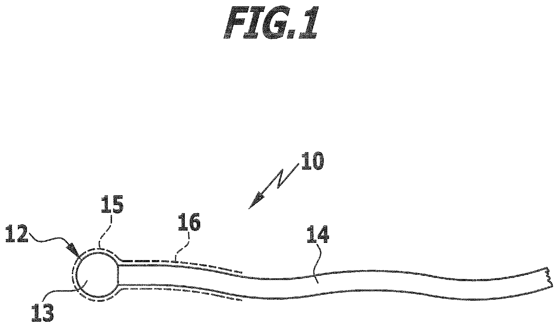

Figur 1 zeigt einen insgesamt mit dem Bezugszeichen 10 versehenen Temperatursensor der vorliegenden Erfindung umfassend ein Sensorelement 12 in Form eines kugelförmigen Rubinkristalls und einen sich daran anschließenden Lichtleiter 14, der optisch mit dem Sensorelement 12 verbunden ist. Bei dieser Ausführungsform des erfindungsgemäßen Temperatursensors 10 ist ein einziger Lichtleiter 14 vorhanden, der sowohl für das Zuführen von Licht mit der Anregungswellenlänge für die Lumineszenz des anregbaren Mediums 13 des Sensorelements 12 dient als auch der Aufnahme und dem Ableiten der von dem Medium 13 abgegebenen Lumineszenz, die durch die Anregungswellenlänge induziert wird. Das Sensorelement 12 kann eine das Medium 13 umgebende Hülle 15 umfassen (in Figur 1 durchbrochen dargestellt), die das Sensorelement mit dem Lichtleiter 14 verbindet. Gegebenenfalls kann der Lichtleiter 14 von einem Hüllmaterial 16 umgeben sein (in Figur 1 durchbrochen dargestellt), das bevorzugt wiederum stoffschlüssig mit der Hülle 15 des Sensorelements verbunden ist.

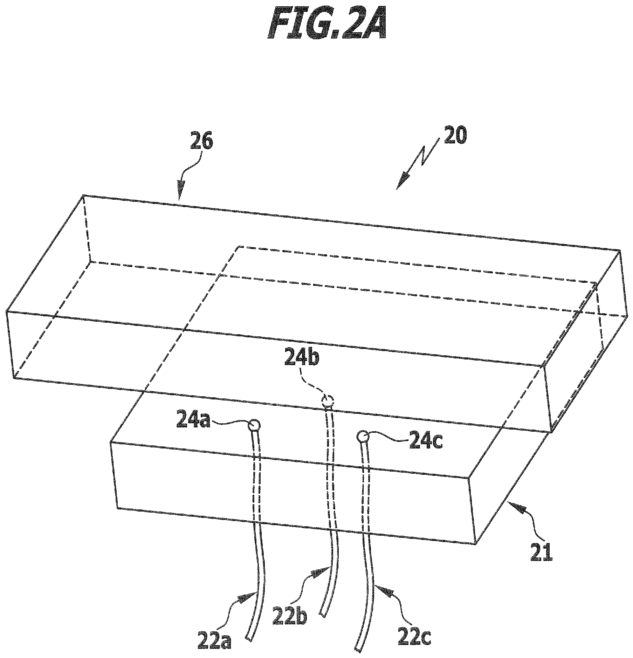

In der Figur 2A ist eine Temperaturmessvorrichtung 20 gezeigt, bei der drei Temperatursensoren 22a, 22b und 22c in einer Halterung 21 gehalten, so dass ihre Sensorelemente 24a, 24b und 24c in einer Dreieck-förmigen Matrix angeordnet sind.

An dem schematisch gezeigten Probenkörper 26 lässt sich nun die Temperatur ortsaufgelöst bestimmen.

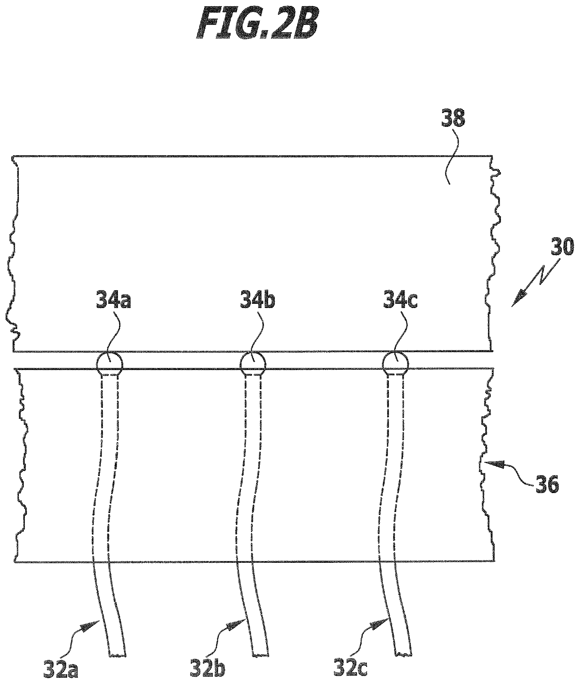

In der Figur 2B ist eine Temperaturmessvorrichtung 30 gezeigt, in der ebenfalls drei Temperatursensoren 32a, 32b, 32c im Abstand voneinander gehalten sind, so dass ihre Sensorelemente 34a, 34b und 34c linear in gleichen Abständen voneinander angeordnet sind.

Gleichzeitig ragen die Sensorelemente 34a, 34b, 34c über die Oberfläche der Halterung 36 hinaus und definieren so einen Abstand zu einem Körper 38, der zusammen mit der Halterung 36 ein Paar Applikatorelemente, beispielsweise für hochfrequenten Strom, bilden kann.

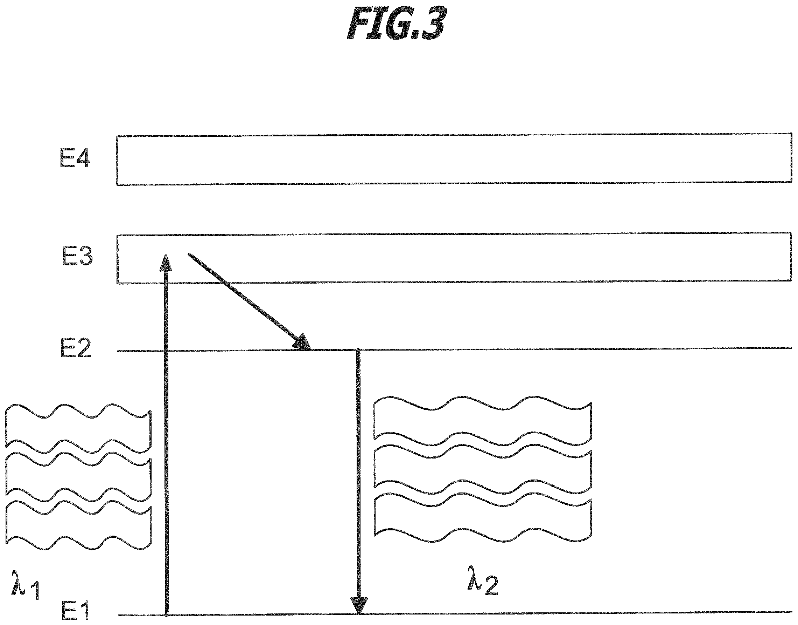

Figur 3 zeigt schematisch das Wirkungsprinzip der Lumineszenzmedien der Sensorelemente der erfindungsgemäßen Temperatursensoren.

Mittels Absorption des Lichts mit einer Anregungswellenlänge λ1 wird das zur Lumineszenz anregbare Medium von einem energetischen Zustand E1 in einen energetisch angeregten Zustand E3 gebracht, von welchem aus das Medium thermisch in den angeregten Zustand E2 relaxiert. Aus dem angeregten Zustand E2 findet unter Emission von Licht der Wellenlänge λ2 eine Rückkehr in den energetischen Zustand E1 statt. Dies soll im Folgenden anhand eines Rubinkristalls als ein zur Lumineszenz anregbares Medium noch näher beschrieben werden.

Bei Rubinkristallen sind Chrom-Ionen verantwortlich für eine Lumineszenz, die hier als Fluoreszenz abgestrahlt wird. Die Chrom-Ionen besitzen optimale spektrale Absorptionsbanden über die Fluoreszenzlicht mit großer Quantenausbeute anregbar ist. Eine der Absorptionsbanden im grünen Spektralbereich ermöglicht eine Anregung des Rubinkristalls als anregbares Medium mit einer Anregungswellenlänge λ1 von ca. 532 nm, während die Emissionswellenlänge λ2 im roten Spektralbereich ca. 694 nm beträgt. Die beobachtete Quantenausbeute ist temperaturabhängig, d.h. das Verhältnis der Zahl von emittierten zu absorbierten Photonen nimmt mit zunehmender Temperatur ab. Diese Temperaturabhängigkeit ist in dem für den in der Medizin interessanten Temperaturbereich von ca. 30 bis ca. 150 °C ausreichend ausgeprägt um hinreichend genaue Temperaturwerte von behandelten Gewebestrukturen zu liefern.

Die beiden Wellenlängen von Anregungslicht und Fluoreszenzlicht liegen so weit auseinander, dass deren Lichtanteile optisch problemlos separiert werden können und die Quantenausbeute der Fluoreszenz mit der Wellenlänge λ2 einfach und genau bestimmbar ist.

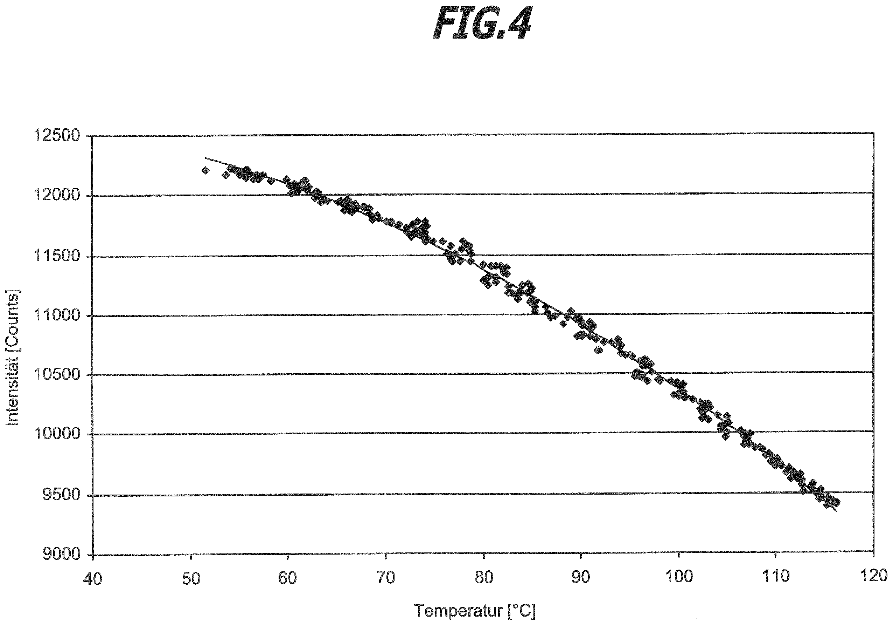

In Figur 4 ist die Fluoreszenzquantenausbeute eines Rubinkristalls gegenüber der Temperatur im Bereich von 50 bis 115 °C aufgetragen, und die dabei erhaltene Kurve kann der Kalibrierung eines erfindungsgemäßen Temperatursensors mit einem Rubinkristall dienen, der bei der Versiegelung von Gewebestrukturen eingesetzt wird.

Es ist ersichtlich, dass hier eine annähernd lineare Abhängigkeit zwischen der Temperatur einerseits und der Fluoreszenzquantenausbeute (hier als Intensität oder Counts einer Fotodiode aufgetragen) besteht. Die Messwerte wurden bei konstanter Anregungslichtintensität an einem in einem Ofen mit geregelter Temperatur platzierten Rubinkristall erhalten.

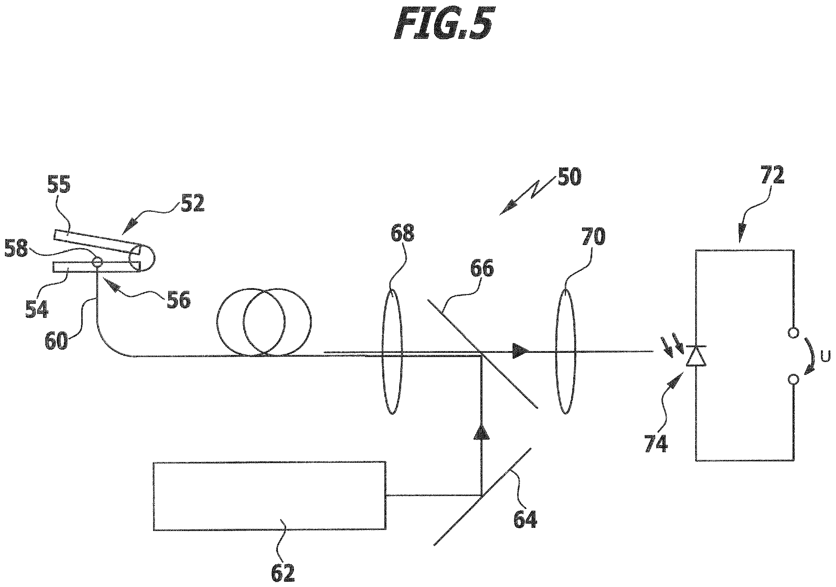

Figur 5 zeigt eine schematische Darstellung eines erfindungsgemäßen medizintechnischen Systems 50.

Das medizintechnische System 50 umfasst hierzu eine Vorrichtung zur Versiegelung und/oder Verbindung von Gewebestrukturen eines Patienten, die mit dem Bezugszeichen 52 versehen ist. In der schematischen Darstellung der Figur 5 ist diese Vorrichtung reduziert auf zwei Applikatorbranchen 54, 55 dargestellt, die schwenkbar miteinander verbunden sind. Der für eine Bestromung der Applikatorbranchen 54, 55 benötigte Generator sowie die Vorrichtung zum Applizieren der Applikatorbranchen 54, 55 an einer Gewebestruktur sind dem Fachmann geläufig und der Einfachheit halber in der Figur 5 weggelassen.

In einer der Applikatorbranchen 54 ist ein erfindungsgemäßer Temperatursensor 56 angeordnet, dessen Sensorelemente 58 so ausgebildet und in der Applikatorbranche 54 so angeordnet ist, dass er gleichzeitig die Funktion eines Abstandhalters übernehmen kann, der verhindert, dass die beiden Applikatorbranchen 54 und 55 direkt miteinander in elektrisch leitenden Kontakt kommen und so ein Kurzschluss zwischen den beiden Branchen 54 und 55 vermieden wird.

Der Temperatursensor 56 weist neben dem Sensorelement 58 einen sich daran anschließenden Lichtleiter 60 auf.

Das medizintechnische System 50 weist des Weiteren eine Lichtquelle in Form eines Lasers 62 auf, dessen Laserstrahl über einen Spiegel 64 und einen dichroitischen Spiegel 66 in den Lichtleiter 60 eingekoppelt wird. Der Laser 62 ist ein Nd:YAG-Laser, dessen Ausgangslicht frequenzverdoppelt wird und so eine Wellenlänge von ca. 532 nm aufweist.

Das bei der Einkopplung verwendete optische System ist in der Figur 5 schematisch durch eine Linse 68 dargestellt. Das Anregungslicht des Lasers 62 wird über den Lichtleiter 60 in das Sensorelement 58 und das dort angeordnete anregbare Medium (im vorliegenden Beispiel wieder ein Rubinkristall) eingestrahlt und generiert hier die weiter oben im Zusammenhang mit den Figuren 3 und 4 beschriebene Fluoreszenz mit einer Wellenlänge von ca. 694 nm, die von dem Rubinkristall isotrop emittiert wird. Der Lichtleiter 60 erfasst von dieser emittierten Strahlung zwar nur einen Bruchteil, jedoch diesen konstant, so dass das von dem Lichtleiter 60 aufgenommene Fluoreszenzlicht des Rubinkristalls des Sensorelements 58 repräsentativ zur Gesamtquantenausbeute ist. Das Fluoreszenzlicht wird von dem Lichtleiter 60 durch das optische System 68 hindurch geleitet und durchdringt den dichroitischen Spiegel 66, der so konzipiert ist, dass er die Anregungswellenlänge von ca. 532 nm reflektiert, während die Wellenlänge von 694 nm durchgelassen wird. Das rote Fluoreszenzlicht wird nach Durchtritt durch den dichroitischen Spiegel 66 über ein weiteres optisches System 70 auf einen Detektor 72 und dessen Photodiode 74 geleitet, wobei das dabei erzeugte Spannungssignal U als Intensität ausgewertet wird.

Der Temperatursensor 56 mit dem Sensorelement 58 und dem Lichtleiter 60 sowie der Laser 62 als Lichtquelle und der Detektor 72 bilden eine Temperaturmessvorrichtung der vorliegenden Erfindung. In bevorzugten Ausführungsformen der erfindungsgemäßen Temperaturmessvorrichtung, wie in Figur 5 als Teil des medizintechnischen Systems 50 dargestellt, dient der Lichtleiter 60 sowohl dem Transport des Lichts mit der Anregungswellenlänge zum Sensorelement 58 als auch dem Transport der Lumineszenz des angeregten Mediums zum Detektor 72. Die optischen Systeme 68 und 70 sowie die Spiegel 64 und 66 schaffen optimierte Lichtwege innerhalb der Temperaturmessvorrichtung.

Dadurch, dass das Sensorelement 58 bzw. der als anregbares Medium verwendete Rubinkristall aus der Oberfläche der Applikatorbranche 54 austretend angeordnet ist, ist er im direkten Kontakt mit dem zwischen den Applikatorbranchen 54, 55 angeordneten Gewebe, so dass eine Temperaturänderung dieses Gewebes sich direkt auf das Sensorelement 58 überträgt. Aufgrund der Temperaturabhängigkeit der Quantenausbeute der Fluoreszenz des Rubinkristalls (vgl. Figur 4) lässt sich somit von den unterschiedlichen Spannungssignalen des Detektors 72 die Temperatur des Gewebes, das zwischen den beiden Applikatorbranchen 54, 55 gehalten wird, ermitteln.

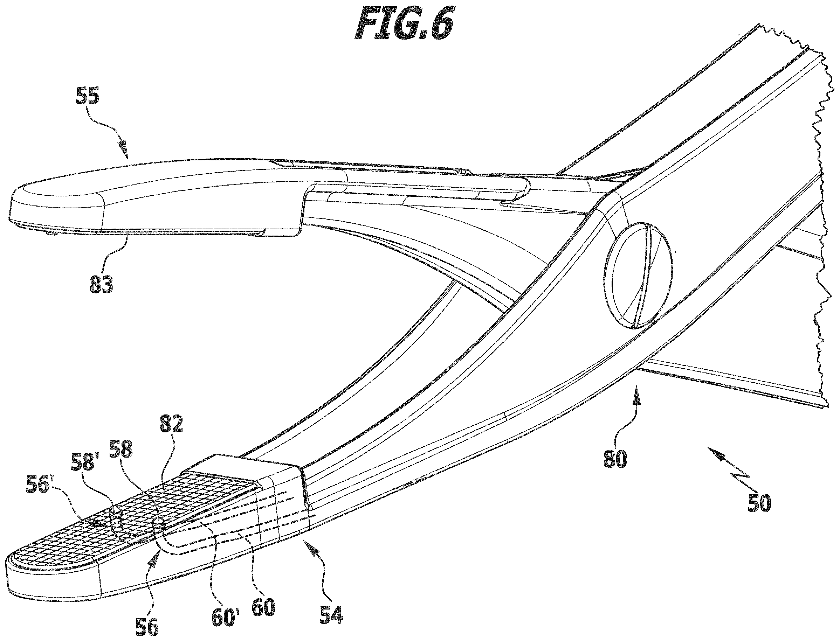

In der Figur 6 sind die beiden Applikatorbranchen 54, 55 nochmals beispielhaft im Detail dargestellt, die elektrisch voneinander isoliert verschwenkbar über einen Gelenkbereich 80 gehalten werden.

Zwei Temperatursensoren 56, 56' sind mit ihren Sensorelementen 58, 58' in der Applikatorbranche 54 angeordnet, wobei die Sensorelemente 58, 58' mit ihrem zur Lumineszenz anregbaren Medium über die Oberfläche der Applikatorbranche 54 hinausragen und so einen Anschlag, d.h. Abstandhalter für die Schließposition der Applikatorbranche 55 bildet. Die beiden Applikatorbranchen 54, 55 sind mit elektrisch leitfähigen und bestrombaren Elektroden 82, 83 ausgerüstet, die vorzugsweise, wie in Figur 6 gezeigt, eine strukturierte Oberfläche, insbesondere eine gitterartig strukturierte Oberfläche, aufweisen, die verhindert, dass zwischen den Applikatorbranchen 54, 55 gehaltenes, zu behandelndes Gewebematerial während der Behandlung verrutschen kann.

## Patentansprüche

1. Medizintechnisches System, umfassend einen oder mehrere Temperatursensor(en), jeweils aufweisend ein Sensorelement (12) mit einem zur Lumineszenz, insbesondere Fluoreszenz, anregbaren Medium (13) und einem optisch mit dem Sensorelement (12) verbundenen Lichtleiter (14) zum Zuführen von Licht zum Medium (13) mit einer Anregungswellenlänge und/oder zum Aufnehmen und Ableiten von Licht mit einer Lumineszenz-Wellenlänge des zur Lumineszenz anregbaren Mediums (13), wobei das medizintechnische System als chirurgisches System, insbesondere als Ultraschall-, Laser- oder elektrochirurgisches System ausgebildet ist,
**dadurch gekennzeichnet,**
**dass** das chirurgische System eine Vorrichtung (82, 83) zum Schneiden, zur Dissektion, zur Koagulation, zur Versiegelung und/oder zur Verbindung von Gewebestrukturen eines Patienten umfasst, wobei die Vorrichtung ein Applikatorwerkzeug mit zwei Applikatorelementen (54, 55) in Form von Applikatorbranchen, umfasst, welche gegeneinander beweglich und aus einer offenen Ruhestellung in eine geschlossene Arbeitsstellung überführbar sind, wobei in mindestens einem der Applikatorelemente der Temperatursensor angeordnet ist und wobei
zumindest einer der Temperatursensoren in zumindest einem der Applikatorelemente (54, 55) so angeordnet ist, dass er während dem Betrieb im direkten Kontakt mit dem zwischen den Applikatorelementen (54, 55) angeordneten Gewebe ist, so dass eine Temperaturänderung dieses Gewebes sich direkt auf das Sensorelement überträgt und wobei
die Temperaturmessung auf Basis einer Temperaturabhängigkeit einer Quantenausbeute einer lumineszenzstrahlung ermittelt wird, die von dem Medium (13) im Sensorelement (12) abgestrahlt wird.

2. Medizintechnisches System nach Anspruch 1, **dadurch gekennzeichnet, dass** die Applikatorelemente (54, 55) einen Abstandhalter aufweisen, der die Applikatorelemente (54, 55) in der Arbeitsstellung in einem vorgegebenen Abstand zueinander hält, wobei der Abstandhalter insbesondere einen Temperatursensor umfasst.

3. Medizintechnisches System nach Anspruch 1 oder 2, **dadurch gekennzeichnet, dass** das System einen Schutzmechanismus umfasst, welcher die Aktivierung des oder der Temperatursensoren blockiert, solange die Applikatorelemente (54, 55) nicht in einer vorgegebenen Applikationsstellung, insbesondere der Arbeitsstellung, sind.

4. Medizintechnisches System nach Anspruch 3, **dadurch gekennzeichnet, dass** der Schutzmechanismus ein Schaltelement, insbesondere einen optischen, elektrischen oder mechanischen Kontaktgeber, aufweist, der die Stellung der Applikatorelemente (54, 55) überwacht.

5. Medizintechnisches System nach Anspruch 3 oder 4, **dadurch gekennzeichnet, dass** der Schutzmechanismus einen separaten Temperatursensor zur Bestimmung der Temperatur mindestens eines der Applikatorelemente (54, 55) umfasst.

6. Medizintechnisches System nach einem der Ansprüche 1 bis 5, **dadurch gekennzeichnet, dass** das System einen HF-Generator, eine Temperaturmessvorrichtung, eine Vorrichtung zur Auswertung eines Temperaturverlaufs und/oder eine Vorrichtung zum Regeln oder Steuern einer Systemfunktion in Abhängigkeit der von dem Temperatursensor gemessenen Temperaturwerte umfasst.

7. Medizintechnisches System nach einem der Ansprüche 1 bis 5, **dadurch gekennzeichnet, dass** die Applikatorelemente (54, 55) einen strukturierten Oberflächenbereich umfassen, der ein Greifen einer Gewebestruktur ermöglicht.

8. Medizintechnisches System nach Anspruch 6, **dadurch gekennzeichnet, dass** der strukturierte Oberflächenbereich eine gitterartige Oberflächenstruktur aufweist.

9. Medizintechnisches System nach einem der Ansprüche 1 bis 7, **dadurch gekennzeichnet, dass** das System eine Dosiervorrichtung für eine Primermaterial, insbesondere ein kollagen- oder gelatinebasierendes Primermaterial, umfasst.

10. Medizintechnisches System nach einem der Ansprüche 1 bis 9, **dadurch gekennzeichnet, dass** die Lumineszenz-Wellenlänge des abgeleiteten Lichtsignals des Temperatursensors dafür angepasst ist, die Basis einer Quantenausbeute-Beobachtung zu bilden, der eine bestimmte Temperatur jeweils zugeordnet oder zuordenbar ist.

11. Medizintechnisches System nach einem der Ansprüche 1 bis 10, **dadurch gekennzeichnet, dass** ein gemeinsamer Lichtleiter (14) zum Zuführen und zum Aufnehmen und Ableiten von Licht mit der Anregungswellenlänge bzw. der Lumineszenz-Wellenlänge vorhanden ist.

12. Medizintechnisches System nach einem der Ansprüche 1 bis 11, **dadurch gekennzeichnet, dass** das Sensorelement (12) direkt mit dem oder den Lichtleiter(n) (14) verbunden ist.

13. Medizintechnisches System nach einem der Ansprüche 1 bis 12, **dadurch gekennzeichnet, dass** das Sensorelement (12) das zur Lumineszenz anregbare Medium (13) in einer das Medium umgebenden Hülle (15) umfasst, die insbesondere starr ausgebildet ist.

14. Medizintechnisches System nach Anspruch 13, **dadurch gekennzeichnet, dass** das zur Lumineszenz anregbare Medium (13) ein fließfähiges Medium ist.

15. Medizintechnisches System nach einem der Ansprüche 1 bis 13, **dadurch gekennzeichnet, dass** das zur Lumineszenz anregbare Medium (13) ein Festkörper, insbesondere ein Rubin, Saphir oder YAG-Materialien enthaltender Festkörper oder ein funktionalisiertes Kunststoffmaterial ist.

16. Medizintechnisches System nach einem der voranstehenden Ansprüche **gekennzeichnet durch** eine die Anregungswellenlänge abstrahlende Lichtquelle (62), insbesondere einen Laser, und einen Lumineszenzdetektor (72), vorzugsweise in Form einer Photozelle.

17. Medizintechnisches System nach einem der vorstehenden Ansprüche, **dadurch gekennzeichnet, dass** mehrere Temperatursensoren in Form einer zwei- oder dreidimensionalen Matrix in einer Halterung (21) in vorgegebenem Abstand zueinander aufgenommen sind.

## Claims

1. Medical system, comprising one or more temperature sensors, each comprising a sensor element (12) having a medium (13) that can be excited to luminescence, in particular fluorescence, and having an optical waveguide (14) that is optically connected to the sensor element (12) and is intended for supplying light having an excitation wavelength to the medium (13) and/or for absorbing and guiding away light having a luminescence wavelength of the medium (13) that can be excited to luminescence, the medical system being designed as a surgical system, in particular as an ultrasound system, laser system or electrosurgical system,
**characterised in that**
the surgical system comprises a device (82, 83) for cutting, dissecting, coagulating, sealing and/or joining tissue structures of a patient, the device comprising an applicator tool having two applicator elements (54, 55) in the form of applicator arms, which can move relative to one another and can be transferred from an open, non-operational position into a closed, working position, the temperature sensor being arranged in at least one of the applicator elements, and at least one of the temperature sensors being arranged in at least one of the applicator elements (54, 55) such that, during operation, said temperature sensor is in direct contact with the tissue arranged between the applicator elements (54, 55), such that a change in temperature of said tissue is transmitted directly to the sensor element, and the temperature being measured on the basis of a temperature dependency of a quantum efficiency of luminescent radiation radiated by the medium (13) in the sensor element (12).

2. Medical system according to claim 1, **characterised in that** the applicator elements (54, 55) comprise a spacer that holds the applicator elements (54, 55) in the working position at a predefined distance from one another, the spacer comprising in particular a temperature sensor.

3. Medical system according to either claim 1 or claim 2, **characterised in that** the system comprises a protective mechanism that prevents activation of the temperature sensor(s) as long as the applicator elements (54, 55) are not in a predefined application position, in particular the working position.

4. Medical system according to claim 3, **characterised in that** the protective mechanism comprises a switch element, in particular an optical, electric or mechanical contact maker, that monitors the position of the applicator elements (54, 55).

5. Medical system according to either claim 3 or claim 4, **characterised in that** the protective mechanism comprises a separate temperature sensor for determining the temperature of at least one of the applicator elements (54, 55).

6. Medical system according to any one of claims 1 to 5, **characterised in that** the system comprises a HF generator, a temperature measurement device, a device for evaluating a temperature curve and/or a device for closed-loop or open-loop control of a system function on the basis of the temperature values measured by the temperature sensor.

7. Medical system according to any one of claims 1 to 5, **characterised in that** the applicator elements (54, 55) comprise a textured surface region that makes it possible to grip a tissue structure.

8. Medical system according to claim 6, **characterised in that** the textured surface region comprises a lattice-like surface texture.

9. Medical system according to any one of claims 1 to 7, **characterised in that** the system comprises a metering device for a primer material, in particular a collagen-based or gelatine-based primer material.

10. Medical system according to any one of claims 1 to 9, **characterised in that** the luminescence wavelength of the guided-away light signal of the temperature sensor is suitable for forming the basis of a quantum efficiency observation to which a particular temperature is or can be assigned in each case.

11. Medical system according to any one of claims 1 to 10, **characterised in that** a common optical waveguide (14) is present for supplying light having the excitation wavelength and/or for absorbing and guiding away light having the luminescence wavelength.

12. Medical system according to any one of claims 1 to 11, **characterised in that** the sensor element (12) is directly connected to the optical waveguide(s) (14).

13. Medical system according to any one of claims 1 to 12, **characterised in that** the sensor element (12) comprises the medium (13) that can be excited to luminescence in a shell (15) that surrounds the medium and is in particular stiff.

14. Medical system according to claim 13, **characterised in that** the medium (13) that can be excited to luminescence is a free-flowing medium.

15. Medical system according to any one of claims 1 to 13, **characterised in that** the medium (13) that can be excited to luminescence is a solid body, in particular a ruby, sapphire or YAG material-containing solid body, or a functionalised plastics material.

16. Medical system according to any one of the preceding claims, **characterised by** a light source (62), in particular a laser, that radiates the excitation wavelength, and a luminescence detector (72), preferably in the form of a photocell.

17. Medical system according to any one of the preceding claims, **characterised in that** a plurality of temperature sensors are held in a holder (21) at a predefined distance from one another in the form of a two-dimensional or three-dimensional matrix.

## Revendications

1. Système technique médical, comprenant un ou plusieurs capteur(s) de température présentant chacun un élément de capteur (12) avec un agent (13) pouvant être excité en luminescence, notamment fluorescence, et un guide d'onde lumineuse (14) relié optiquement à l'élément de capteur (12) pour l'amenée de lumière à l'agent (13) avec une longueur d'onde d'excitation et/ou pour capter et évacuer de la lumière avec une longueur d'onde de luminescence de l'agent (13) pouvant être excité en luminescence, le système technique médical étant réalisé en tant que système chirurgical, notamment en tant que système chirurgical à ultrasons, à laser ou électrique,
**caractérisé en ce que**
le système chirurgical comprend un dispositif (82, 83) pour la coupe, pour la dissection, pour la coagulation, pour le scellement et/ou pour assurer la liaison ou suture de structures tissulaires d'un patient,
le dispositif comprend un outil applicateur avec deux éléments d'applicateur (54, 55) qui se présentent sous la forme de branches d'applicateur, sont mobiles l'un par rapport à l'autre et peuvent être transférés d'une position ouverte de repos à une position fermée de travail,
dans au moins l'un des éléments d'applicateur est agencé le capteur de température, et
au moins l'un des capteurs de température est agencé dans au moins l'un des éléments d'applicateur (54, 55) de manière telle, que, pendant le service, il soit en contact direct avec les tissus se trouvant entre les éléments d'applicateur (54, 55), de sorte qu'une variation de température de ces tissus soit directement transmise à l'élément de capteur, et
la mesure de température est déterminée sur la base d'une relation de dépendance de la température d'un rendement quantique d'un rayonnement de luminescence, qui est rayonné par l'agent (13) dans l'élément de capteur (13).

2. Système technique médical selon la revendication 1, **caractérisé en ce que** les éléments d'applicateur (54, 55) présentent une entretoise d'espacement, qui maintient les éléments d'applicateur (54, 55) à une distance prescrite, dans la position de travail, l'entretoise d'espacement comprenant notamment un capteur de température.

3. Système technique médical selon la revendication 1 ou la revendication 2, **caractérisé en ce que** le système comprend un mécanisme de protection, qui bloque l'activation du ou des capteurs de température aussi longtemps que les éléments d'applicateur (54, 55) ne sont pas dans une position d'application prescrite, notamment la position de travail.

4. Système technique médical selon la revendication 3, **caractérisé en ce que** le mécanisme de protection comprend un élément de commutation, notamment un contacteur optique, électrique ou mécanique, qui surveille la position des éléments d'applicateur (54, 55) .

5. Système technique médical selon la revendication 3 ou la revendication 4, **caractérisé en ce que** le mécanisme de protection comprend un capteur de température supplémentaire pour déterminer la température d'au moins l'un des éléments d'applicateur (54, 55).

6. Système technique médical selon l'une des revendications 1 à 5, **caractérisé en ce que** le système comprend un générateur HF, un dispositif de mesure de température, un dispositif pour assurer le traitement des données d'une loi de variation de température, et/ou un dispositif pour réguler ou commander une fonction du système, en corrélation avec les valeurs de température mesurées par le capteur de température.

7. Système technique médical selon l'une des revendications 1 à 5, **caractérisé en ce que** les éléments d'applicateur (54, 55) comportent une zone de surface structurée, qui permet la saisie ou préhension d'une structure tissulaire.

8. Système technique médical selon la revendication 6, **caractérisé en ce que** la zone de surface structurée présente une structure de surface en forme de treillis.

9. Système technique médical selon l'une des revendications 1 à 7, **caractérisé en ce que** le système comprend un dispositif de dosage pour un matériau primaire, notamment un matériau primaire à base de collagène ou de gélatine.

10. Système technique médical selon l'une des revendications 1 à 9, **caractérisé en ce que** la longueur d'onde de luminescence du signal lumineux évacué du capteur de température est adapté à former la base d'une observation de rendement quantique auquel est associée ou peut être associée une température déterminée.

11. Système technique médical selon l'une des revendications 1 à 10, **caractérisé en ce qu'**il est prévu un guide d'onde lumineuse (14) commun pour amener et capter et évacuer de la lumière présentant une longueur d'onde d'excitation et respectivement une longueur d'onde de luminescence.

12. Système technique médical selon l'une des revendications 1 à 11, **caractérisé en ce que** l'élément de capteur (12) est relié directement au ou aux guide(s) d'onde lumineuse (14).

13. Système technique médical selon l'une des revendications 1 à 12, **caractérisé en ce que** l'élément de capteur (12) comprend l'agent (13) pouvant être excité en luminescence, dans une enveloppe (15) qui entoure l'agent et est notamment d'une configuration rigide.

14. Système technique médical selon la revendication 13, **caractérisé en ce que** l'agent (13) pouvant être excité en luminescence est un agent fluide.

15. Système technique médical selon l'une des revendications 1 à 13, **caractérisé en ce que** l'agent (13) pouvant être excité en luminescence est un corps solide, notamment un corps solide renfermant du rubis, du saphir ou des matériaux YAG, ou bien une matière plastique fonctionnalisée.

16. Système technique médical selon l'une des revendications précédentes, **caractérisé par** une source de lumière (62) rayonnant la longueur d'onde d'excitation, notamment un laser, et par un détecteur de luminescence (72), de préférence sous la forme d'une cellule photoélectrique.

17. Système technique médical selon l'une des revendications précédentes, **caractérisé en ce que** plusieurs capteurs de température sont logés à une distance prédéterminée les uns des autres dans un support (21), sous la forme d'une matrice à deux ou trois dimensions.
